# EUROPEAN PATENT APPLICATION

(11) **EP 2 489 359 A1**
(43) Date of publication of application: **22.08.2012**
(21) Application number: 10823369.3
(22) Date of filing: 12.10.2010
(51) Int. Cl.: A61K 38/00, A23J 3/08, A23K 1/08, A23K 1/16, A23L 1/305, A23L 2/38, A23L 2/66, A61P 3/04, A61P 3/06

(54) **FAT ACCUMULATION SUPPRESSOR**

(30) Priority: 13.10.2009 JP 2009236588
(71) Applicant: Megmilk Snow Brand Co., Ltd., Sapporo-shi Hokkaido 0650043 (JP)
(72) Inventor: NAITO, Hisashi, Inzai-shi Chiba 270-1606 (JP); MIURA, Susumu, Sapporo-shi Hokkaido 065-0043 (JP)
(74) Representative: Wakerley, Helen Rachael
(86) International application number: PCT/JP2010/067845
(87) International publication number: WO 2011/046101

(57) **Abstract**

A fat accumulation suppressor comprising as an active ingredient a whey protein hydrolysate is provided. The hydrolysate has a molecular weight range of no higher than 10 kDa and a main peak of molecular weight at 200 Da to 3 kDa; an APL (average peptide chain length) of 2 to 8; a free amino acid content of 20% or lower; a branched chain amino acid content of 20% or higher; and an antigenicity of 1/100,000 or lower relative to β-lactoglobulin.

## Description

### Technical Field

The present invention relates to a fat accumulation suppressor (an agent for suppressing fat accumulation) comprising as an active ingredient a whey protein hydrolysate characterized by reduced bitterness and excellent stability and safety.
The present invention also relates to food and beverage products for suppressing fat accumulation, a nutritional composition for suppressing fat accumulation, and animal feed for suppressing fat accumulation, comprising as an active ingredient a whey protein hydrolysate characterized by reduced bitterness and excellent stability and safety.
The fat accumulation suppressor of the present invention is effective for preventing body fat accumulation caused by metabolic slowdown related to ageing or senescence, and moreover, it is characterized by reduced bitterness and excellent stability and safety.

### Background Art

Ageing and senescence are known to cause a variety of physiological changes (such as accumulation of fats due to reduced basal metabolic rates, withering of skeletal muscles, and decalcification of bones) in the body of an organism. In particular, obesity caused by fat accumulation is problematic because it can lead to increased likelihood of myocardial infarction and death as well as to lowered Quality of Life (QOL) (Non-Patent Documents 1 and 2). Thus, researches on methods for improving obesity have been actively conducted in order to reduce myocardial infarctions and mortality (Non-Patent Documents 3 and 4).
Improved diet and regular and moderate exercise are some of the ways to improve obesity but they usually require certain levels of patience and pain. Some food components have been investigated for a possible effect of improving obesity. For example, consumption of soybean peptides has been reported to have a fat-reducing effect (Patent Document 1).

Cow milk and dairy products are often recognized as a cause of food allergy In particular, the whey proteins that are not found in mother's milk of humans are suspected allergens. Thus, methods for reducing the allergenic potentials by hydrolyzing the whey proteins with enzymes have been known, as disclosed in Patent Document 2, for example.
In particular, it has been shown that the antigenicity of the whey protein hydrolysate produced according to the method of Patent Document 2 is at most 1/10,000 of that of β-lactoglobulin and at most 1/10,000 of that of the whey proteins as measured by the Inhibition ELISA method (Non-Patent Document 5).
The said whey protein hydrolysate was also shown to have a muscle-building effect when it is taken orally and thus drawing attention for being a material characterized by both the low-allergenicity and the functionality

### Prior Art Documents

### Patent Documents

Patent Document 1: JP 2002-20312 A
Patent Document 2: WO 2008/111562 A

### Non-Patent Documents

Non-Patent Document 1: Obesity in Old Age, I. M. Chapman, Obesity and Metabolism, Front. Horm. Res. Basel, Karger, 2008, Vol.36, pp 97-106
Non-Patent Document 2: Obesity as an independent risk factor for cardiovascular disease, Circulation 1983, Vol. 67, pp 968-977
Non-Patent Document 3: Primary prevention of cardiovascular disease, Acta Med Croatica 2009, Vol. 63, pp 71-74
Non-Patent Document 4: Prevention of excess gain, Int. J. Obes. (Lond) 2009, in press.
Non-Patent Document 5: Proceedings of the Japanese Society of Pediatric Allergy and Clinical Immunology, 1, 36 (1987)

### Summary of the Invention

### Problem to Be Solved by the Invention

The known method of suppressing obesity by ingestion of the soybean peptides does exhibit an effect, but problematically the soybean peptides have the characteristic bitterness, and moreover, they cause cloudiness when dispersed in water, which limits their applicability in food, beverage or other products, especially in the products that require transparency.
The whey protein hydrolysate obtained by hydrolysis of the whey proteins of cow milk, on the other hand, has low allergenicity has little bitterness, and does not precipitate, and is therefore suitable for use in food and beverage products, although there have been no reports as to whether it has any effect for suppressing fat accumulation.

Thus, in the view of the problems mentioned above, an object of the present invention is to provide a fat accumulation suppressor that provides an effect of suppressing fat accumulation and that does not suffer the shortcomings of tasting bitter, causing cloudiness or forming precipitation.

### Means of Solving the Problem

The present inventors have conducted extensive research and found that the whey protein hydrolysate obtained by hydrolysis of the whey proteins from cow milk has a potent effect of suppressing fat accumulation. This finding has led to the completion of the present invention.
Thus, the present invention provides a fat accumulation suppressor, and food and beverage products for suppressing fat accumulation, a nutritional composition for suppressing fat accumulation, and animal feed for suppressing fat accumulation, comprising the said fat accumulation suppressor.

The present invention comprises the following.
(1) A fat accumulation suppressor comprising as an active ingredient a whey protein hydrolysate having the following characteristics:
   1. a molecular weight range of no higher than 10 kDa with a main peak of molecular weight at 200 Da to 3 kDa;
   2. an APL (average peptide chain length) of 2 to 8;
   3. a free amino acid content of 20% or lower;
   4. a branched chain amino acid content of 20% or higher; and
   5. an antigenicity of 1/100,000 or lower relative to β-lactoglobulin.
(2) The fat accumulation suppressor according to (1), wherein the whey protein hydrolysate is obtainable by a process comprising:
   a step of hydrolyzing whey proteins with a use of a thermo-resistant protease enzyme while the whey proteins are heat-denatured at a temperature of 50 to 70°C and a pH of 6 to 10; and
   a step of inactivating the enzyme by heating.
(3) The fat accumulation suppressor according to (1), wherein the whey protein hydrolysate is obtainable by a process comprising:
   a step of preliminarily hydrolyzing whey proteins with a use of a protease enzyme at a temperature of 20 to 55°C and a pH of 6 to 10;
   a step of increasing the temperature to 50 to 70°C and hydrolyzing the whey proteins that remained unhydrolyzed in the preliminary hydrolysis step with a use of a thermo-resistant protease enzyme while the unhydrolyzed whey proteins are heat-denatured at a temperature of 50 to 70°C and a pH of 6 to 10; and
   a step of inactivating the enzyme by heating.
(4) The fat accumulation suppressor according to (1), wherein the whey protein hydrolysate is obtainable by a process comprising:
   a step of preliminarily hydrolyzing whey proteins with a use of a protease enzyme at a temperature of 20 to 55°C and a pH of 6 to 10;
   a step of increasing the temperature to 50 to 70°C and hydrolyzing the whey proteins that remained unhydrolyzed in the preliminary hydrolysis step with a use of a thermo-resistant protease enzyme while the unhydrolyzed whey proteins are heat-denatured at a temperature of 50 to 70°C and pH of 6 to 10;
   a step of inactivating the enzyme by heating;
   a step of subjecting the inactivated reaction mixture to an ultrafiltration treatment with an ultrafiltration membrane having a molecular weight cut-off of 1 to 20 kDa; and
   a step of subjecting a flow-through fraction from said ultrafiltration treatment to a microfiltration treatment with a microfiltration membrane having a molecular weight cut-off of 100 to 500 Da and collecting a non-flow-through fraction from said microfiltration treatment.
(5) A food or beverage product for suppressing fat accumulation, a nutritional composition for suppressing fat accumulation, or animal feed for suppressing fat accumulation, comprising the fat accumulation suppressor according to any of (1) to (4).
(6) A method of producing a fat accumulation suppressor, comprising:
   a step of hydrolyzing whey proteins with a use of a thermo-resistant protease enzyme while the whey proteins are heat-denatured at a temperature of 50 to 70°C and a pH of 6 to 10; and
   a step of inactivating the enzyme by heating.
(7) The method according to (6), further comprising, prior to said two steps,
   a step of preliminarily hydrolyzing the whey proteins with a use of a protease enzyme at a temperature of 20 to 55°C and a pH of 6 to 10.
(8) The method according to (6) or (7), further comprising, subsequent to the step of inactivating the enzyme,
   a step of subjecting the inactivated reaction mixture to an ultrafiltration treatment with an ultrafiltration membrane having a molecular weight cut-off of 1 to 20 kDa; and
   a step of subjecting a flow-through fraction from said ultrafiltration treatment to a microfiltration treatment with a microfiltration membrane having a molecular weight cut-off of 100 to 500 Da and collecting a non-flow-through fraction from said microfiltration treatment.
(9) A method of suppressing fat accumulation in a subject, comprising administering to the subject 10 g/day or more of a whey protein hydrolysate having the following characteristics:
   1. a molecular weight range of no higher than 10 kDa with a main peak of molecular weight at 200 Da to 3 kDa;
   2. an APL (average peptide chain length) of 2 to 8;
   3. a free amino acid content of 20% or lower;
   4. a branched chain amino acid content of 20% or higher; and
   5. an antigenicity of 1/100,000 or lower relative to β-lactoglobulin.

The present invention also comprises the following:
(A) A method of suppressing fat accumulation in a subject, comprising administering to the subject a whey protein hydrolysate having the following characteristics:
   1. a molecular weight range of no higher than 10 kDa with a main peak of molecular weight at 200 Da to 3 kDa;
   2. an APL (average peptide chain length) of 2 to 8;
   3. a free amino acid content of 20% or lower;
   4. a branched chain amino acid content of 20% or higher; and
   5. an antigenicity of 1/100,000 or lower relative to β-lactoglobulin.
(B) The method according to (A), wherein the whey protein hydrolysate is obtainable by a process comprising:
   a step of hydrolyzing whey proteins with a use of a thermo-resistant protease enzyme while the whey proteins are heat-denatured at a temperature of 50 to 70°C and a pH of 6 to 10; and
   a step of inactivating the enzyme by heating.
(C) The method according to (A), wherein the whey protein hydrolysate is obtainable by a process comprising:
   a step of preliminarily hydrolyzing whey proteins with a use of a protease enzyme at a temperature of 20 to 55°C and a pH of 6 to 10;
   a step of increasing the temperature to 50 to 70°C and hydrolyzing the whey proteins that remained unhydrolyzed in the preliminary hydrolysis step with a use of a thermo-resistant protease enzyme while the unhydrolyzed whey proteins are heat-denatured at a temperature of 50 to 70°C and a pH of 6 to 10; and
   a step of inactivating the enzyme by heating.
(D) The method according to (A), wherein the whey protein hydrolysate is obtainable by a process comprising:
   a step of preliminarily hydrolyzing whey proteins with a use of a protease enzyme at a temperature of 20 to 55°C and a pH of 6 to 10;
   a step of increasing the temperature to 50 to 70°C and hydrolyzing the whey proteins that remained unhydrolyzed in the preliminary hydrolysis step with a use of a thermo-resistant protease enzyme while the unhydrolyzed whey proteins are heat-denatured at a temperature of 50 to 70°C and a pH of 6 to 10;
   a step of inactivating the enzyme by heating;
   a step of subjecting the inactivated reaction mixture to an ultrafiltration treatment with an ultrafiltration membrane having a molecular weight cut-off of 1 to 20 kDa; and
   a step of subjecting a flow-through fraction from said ultrafiltration treatment to a microfiltration treatment with a microfiltration membrane having a molecular weight cut-off of 100 to 500 Da and collecting a non-flow-through fraction from said microfiltration treatment.

The present invention also comprises the following:
(E) A method of improving obesity caused by accumulation of body fat in a subject, comprising administering to the subject a whey protein hydrolysate having the following characteristics:
   1. a molecular weight range of no higher than 10 kDa with a main peak of molecular weight at 200 Da to 3 kDa;
   2. an APL (average peptide chain length) of 2 to 8;
   3. a free amino acid content of 20% or lower;
   4. a branched chain amino acid content of 20% or higher; and
   5. an antigenicity of 1/100,000 or lower relative to β-lactoglobulin.
(F) The method according to (E), wherein the whey protein hydrolysate is obtainable by a process comprising:
   a step of hydrolyzing whey proteins with a use of a thermo-resistant protease enzyme while the whey proteins are heat-denatured at a temperature of 50 to 70°C and a pH of 6 to 10; and
   a step of inactivating the enzyme by heating.
(G) The method according to (E), wherein the whey protein hydrolysate is obtainable by a process comprising:
   a step of preliminarily hydrolyzing whey proteins with a use of a protease enzyme at a temperature of 20 to 55°C and a pH of 6 to 10;
   a step of increasing the temperature to 50 to 70°C and hydrolyzing the whey proteins that remained unhydrolyzed in the preliminary hydrolysis step with a use of a thermo-resistant protease enzyme while the unhydrolyzed whey proteins are heat-denatured at a temperature of 50 to 70°C and a pH of 6 to 10; and
   a step of inactivating the enzyme by heating.
(H) The method according to (E), wherein the whey protein hydrolysate is obtainable by a process comprising:
   a step of preliminarily hydrolyzing whey proteins with a use of a protease enzyme at a temperature of 20 to 55°C and a pH of 6 to 10;
   a step of increasing the temperature to 50 to 70°C and hydrolyzing the whey proteins that remained unhydrolyzed in the preliminary hydrolysis step with a use of a thermo-resistant protease enzyme while the unhydrolyzed whey proteins are heat-denatured at a temperature of 50 to 70°C and a pH of 6 to 10;
   a step of inactivating the enzyme by heating;
   a step of subjecting the inactivated reaction mixture to an ultrafiltration treatment with an ultrafiltration membrane having a molecular weight cut-off of 1 to 20 kDa; and
   a step of subjecting a flow-through fraction from said ultrafiltration treatment to a microfiltration treatment with a use of a microfiltration membrane having a molecular weight cut-off of 100 to 500 Da and collecting a non-flow-through fraction from said microfiltration treatment.

### Effects of the Invention

The fat accumulation suppressor of the present invention provides a marked effect of suppressing fat accumulation.

### Embodiments of the Invention

Below, the present invention will be explained in detail.

### [Active ingredient]

The whey protein hydrolysate used as an active ingredient in the fat accumulation suppressor of the present invention has the following properties:
1. a molecular weight range of no higher than 10 kDa with a main peak of molecular weight at 200 Da to 3 kDa;
2. an APL (average peptide chain length) of 2 to 8;
3. a free amino acid content of 20% or lower;
4. a branched chain amino acid content of 20% or higher; and
5. an antigenicity of 1/100,000 or lower relative to β-lactoglobulin.
Thus, antigenicity of the whey protein hydrolysate, which is an active ingredient in the present invention, is 1/100,000 or lower as compared to β-lactoglobulin, and 1/100,000 or lower as compared to the whey proteins, and therefore the hydrolysate is extremely safe in terms of suppression of food allergy.
Moreover, the whey protein hydrolysate makes transparent solutions, and its bitterness score is only about 2. Thus, the whey protein hydrolysate is free from the limitations related to taste or appearance when it is used as a fat accumulation suppressor. In particular, the whey protein hydrolysate can be added at high concentrations in fat accumulation suppressors that require transparent appearances. It is also possible to improve water solubility of the whey protein hydrolysate by subjecting it to ultrafiltration (UF) and/or microfiltration (MF) treatments.

It is also possible to provide a food or beverage product for suppressing fat accumulation, a nutritional composition for suppressing fat accumulation, or animal feed for suppressing fat accumulation, which comprises the said fat accumulation suppressor as an active ingredient, provides an effect of suppressing fat accumulation, and is characterized by excellent safeness.
Since the fat accumulation suppressor of the present invention is made from whey proteins, its production is easy, simple and economical.

### [Production method]

The whey protein hydrolysate to be contained in the fat accumulation suppressor of the present invention can be obtained by adding a thermo-resistant protease enzyme to whey proteins at a temperature of 50 to 70°C and a pH of 6 to 10 to hydrolyze the whey proteins while the whey proteins are heat-denatured, and then inactivating the enzyme by heating. The efficiency of the overall hydrolysis may be further improved by first hydrolyzing the whey proteins with a use of a protease enzyme at a temperature of 20 to 55°C and a pH of 6 to 10 and then, without lowering the temperature, immediately proceeding to the hydrolysis under the conditions described in the preceding sentence.

The effect of suppressing fat accumulation may be further enhanced if the whey protein hydrolysate prepared as above is concentrated by an ultrafiltration (UF) membrane having a molecular weight cut-off of 1 kDa to 20 kDa, more preferably 2 kDa to 10 kDa, and/or a microfiltration (MF) membrane having a molecular weight cut-off of 100 Da to 500 Da, more preferably 150 Da to 300 Da. These filtration treatments may also be helpful for reducing bitterness and for increasing transparency of the solution.

The whey in the present invention may refer to whey of a mammalian animal such as cow, buffalo, goat and human, or concentrates, powders, or purified proteins obtained from the said whey. The whey is subjected to an enzymatic treatment in the form of an aqueous solution.
The said solution is adjusted to a pH 6 to 10. Usually, pH of a whey solution is automatically in this pH range and no additional adjustment of pH is needed. However, if needed, adjustment of the solution to a pH 6 to 10 may be carried out by an acidic solution such as hydrochloric acid solution, citric acid solution and lactic acid solution, or by a basic solution such as sodium hydroxide solution, potassium hydroxide solution, and sodium phosphate solution. When the temperature of the solution is increased to 50 to 70°C by heating, the thermo-resistant protease enzyme is more preferably added before the heating than after the heating, so that the enzymatic hydrolysis takes place even during the temperature rise, which would increase overall efficiency of the hydrolysis.

A common protein-hydrolyzing enzyme, protease, has an optimal reaction temperature of 40°C or lower, but thermo-resistant protease enzymes have an optimal reaction temperature of 45°C or higher. Any thermo-resistant protease enzymes having such an optimal reaction temperature may be used in the present invention. Examples of such thermo-resistant protease enzymes include papain, Protease S (brand name), Proleather (brand name), Thermoase (brand name), Alcalase (brand name), and Protin A (brand name). A preferable thermo-resistant protease enzyme will retain 10% or more of the activity after it is heated at 80°C for 30 minutes. Hydrolysis may be carried out more efficiently if two or more enzymes are used in combination. The hydrolysis reaction is preferably carried out for 30 minutes to 10 hours.

The enzyme is finally inactivated by heating up the reaction solution. Inactivation of the enzyme may be carried out by heating the reaction solution at 100°C or higher for 10 seconds or longer.
After the enzyme is inactivated, the reaction solution is centrifuged, and the supernatant is collected and dried to a powder form. The precipitate formed during the centrifugation is preferably removed because its allergenic potential has been reduced to a lesser extent than the supernatant. However, if antigenicity of the precipitate is not problematic, the entire reaction solution may be dried directly and used.

It has been established that the antigenicity of the whey protein hydrolysate obtained by the said method is one 100,000th or less of the antigenicity of β-lactoglobulin, and one 100,000th or less of the antigenicity of the whey proteins, as measured by the Inhibition ELISA method. Thus, the whey protein hydrolysate is extremely safe. Moreover, the whey protein hydrolysate makes transparent solutions, and its bitterness score is only about 2, which circumvents the color- and flavor-related problems when it is used in the products. Transparency and bitterness were evaluated as follows.

Evaluation method of transparency: 1% whey protein hydrolysate solution was prepared and the absorbance at 650 nm was measured.

Evaluation method of bitterness: 10% whey protein hydrolysate solution was prepared, and the bitterness was evaluated in comparison to a bitter substance quinine hydrochloride. As shown in Table 1, a bitterness score of 2 or lower is acceptable in the use for food and beverage products.

APL of the obtained whey protein sample may be measured by HPLC or other suitable methods.

**[Table 1]**

| Quinine hydrochloride concentration | Bitterness score |
|---|---|
| 0.004% | 1 (weak) |
| 0.010% | 2 |
| 0.020% | 3 (strong) |

### [Applications]

The whey protein hydrolysate of the present invention may be used directly as a fat accumulation suppressor, or first formulated into powder, granule, tablet, capsule, drinkable, or any other preparations according to the conventional methods. The whey protein hydrolysate that has been subjected to a further ultrafiltration (UF) or microfiltration (MF) membrane treatment may also be used directly as a fat accumulation suppressor. It may also be dried first or formulated into any of the preparations according to the conventional methods.
Moreover, the said preparations may also be added to nutritional compositions, various food products such as yogurts, beverages, dairy beverages and wafers, animal feeds, supplements, and the likes.

There is no particular limitation as to the amount of the protein hydrolysate added to the food products, nutritional compositions, and animal feeds for suppressing fat accumulation. However, it is preferable to design the product in such a way that it contains an amount of the whey protein hydrolysate sufficient for allowing an adult to take 10 g or more, preferably 20 g or more, of the whey protein hydrolysate per day.
The fat accumulation suppressor of the present invention may be formulated into an oral administration preparation by adding thereto appropriate auxiliary ingredients.

Below, the present invention will be explained in further details by Examples and Comparative Examples, but the present invention is not limited to these examples.

### [Example 1]

To 1 L of a 10% whey protein aqueous solution, 50 U of papain and 150 U of Proleather (Amano Enzyme Inc.) per gram of the whey proteins were added, the pH was adjusted to 8, and enzymatic hydrolysis was carried out at 55°C for 6 hours as the whey proteins were being denatured. The enzymes were inactivated by heating the reaction solution at 100°C for at least 15 seconds. Following a centrifugation, the supernatant was collected and dried to give a whey protein hydrolysate (Example 1 product).
The obtained whey protein hydrolysate (Example 1 product) had a molecular weight range of no higher than 10 kDa, the main peak of molecular weight was at 1.3 kDa, the APL was 7.2, and the free amino acid content relative to the total mass of the product was 18.9%.
Antigenicity of the whey protein hydrolysate was 1/100,000 or lower relative to β-lactoglobulin as measured by the inhibition ELISA method, and the yield (i.e. percentage of the dry weight of the supernatant obtained after a centrifugation of the reaction solution relative to the dry weight of the total reaction solution) was 80.3%. The bitterness score was 2.
The whey protein hydrolysate (Example 1 product) thus obtained may be directly used as a fat accumulation suppressor of the present invention.

### [Example 2]

To 1 L of a 10% whey protein aqueous solution, 50 U of papain and 150 U of Proleather per gram of the whey proteins were added, and enzymatic hydrolysis was carried out at a pH of 8 and a temperature of 50°C for 3 hours. The temperature was then raised to 55°C and maintained at 55°C for 3 hours to continue the enzymatic hydrolysis while the whey proteins were being denatured. The enzymes were inactivated by heating at 100°C for at least 15 seconds. The reaction solution was treated with a UF membrane having a molecular weight cut-off of 10 kDa (STC corporation) and then a MF membrane having a molecular weight cut-off of 300 Da (STC corporation). The concentrate (non-flow-through) fraction was collected and dried to give a whey protein hydrolysate (Example 2 product).
The obtained whey protein hydrolysate (Example 2 product) had a molecular weight range of no higher than 10 kDa, the main peak of molecular weight was at 500 Da, the APL was 3.0, and the free amino acid content relative to the total mass of the product was 15.2%.
Antigenicity of the whey protein hydrolysate was 1/100,000 or lower relative to β-lactoglobulin as measured by the inhibition ELISA method, and the yield was 65.4%. The bitterness score was 2. The whey protein hydrolysate (Example 2 product) thus obtained may be directly used as a fat accumulation suppressor of the present invention.

### [Comparative Example 1]

120 g of whey protein was dissolved in 1,800 ml of purified water, and the pH was adjusted to 7.0 with a 1M caustic soda solution. The sample was then sterilized by heating at 60°C for 10 minutes. The temperature was maintained at 45°C, 20 g of Amano A (Amano Enzyme Inc.) was added, and the enzymatic reaction was continued for 2 hours. The enzyme was inactivated by heating at 80°C for 10 minutes. After a freeze-drying treatment, a whey protein hydrolysate (Comparative Example 1 product) was obtained. The hydrolysis rate of the obtained whey protein hydrolysate was 18%, and the yield was 80.6%.

### [Comparative Example 2]

120 g of whey protein was dissolved in 1,800 ml of purified water, and the pH was adjusted to 7.0 with a 1M caustic soda solution. The mixture was then sterilized by heating at 60°C for 10 minutes. The temperature was maintained at 45°C, 20 g of Amano A (Amano Enzyme Inc.) was added, and the enzymatic reaction was continued for 8 hours. The enzyme was inactivated by heating at 80°C for 10 minutes. After a freeze-drying treatment, a whey protein hydrolysate (Comparative Example 2 product) was obtained. The hydrolysis rate of the obtained whey protein hydrolysate was 30%, and the yield was 80.6%.

### [Test Example 1]

### Transparency test

A 1% aqueous solution of each of the protein hydrolysates from Examples 1 and 2 and Comparative Examples 1 and 2 was prepared, and the absorbance at 650 nm was measured. The results are shown in Table 2.

**[Table 2]**

| Sample | Absorbance (650 nm) |
|---|---|
| Example 1 | 0.008 |
| Example 2 | 0.004 |
| Comparative Example 1 | 0.064 |
| Comaprative Example 2 | 0.018 |

The whey protein hydrolysates of Examples 1 and 2 had high transparency as indicated by the low absorbance. On the other hand, the samples from Comparative Examples showed higher absorbance and lower transparency compared to Examples 1 and 2. Example 2 product which had undergone a membrane treatment had lower absorbance and superior transparency than Example 1 product.

### [Test Example 2]

### Fat accumulation suppression test

Twenty-week-old female SAM-P rats (purchased from Japan SLC, Inc.) were divided into 5 groups in such a way that the average weights of the rats were similar between the groups (in each group, n=6 to 10; 144±6.8 g), and used in the test.
The rats were fed with an AIN93 (standard feed)-based feed in which casein protein was replaced by un-hydrolyzed whey protein, Example 1 product, Example 2 product, or Comparative Example 1 product. As a control for fattiness, 20-week-old female SAM-R rats (n=8; 143±5.4 g) were fed with the AIN93 standard feed. The rats in each group were allowed free access to the food, but the amount of the food consumed was recorded every 2 or 3 days.
When the rats reached the age of 45 weeks, they were anesthetized with pentobarbital sodium (50 mg/kg) and their white adipose tissues (WAT) were extracted and subjected to white adipose tissue weight measurements. Relative white adipose tissue weights per total body weight are shown in Table 3.

**[Table 3]**

| | WAT weight/body weight (mg/g) |
|---|---|
| Control (AIN93) | 10±8.2 |
| Standard feed (AIN93) | 38±8.5 b |
| Whey protein | 42±6.5 b |
| Example 1 | 21±8.7 cd |
| Example 2 | 20±7.3 cd |
| Comparative Example 1 | 40±6.7 b |

| | |
|---|---|
| b : significantly different from the control group (p<0.05) c : significantly different from the standard feed group (p<0.05) d : significantly different from the whey protien group (p<0.05) | |

The standard feed group showed a significant increase in the white adipose tissue weight per body weight, compared to the control group. This indicates that the SAM-P female rats, which are senescence accelerated rodents, had a reduced basal metabolic rate compared to the SAM-R rats. The whey protein group and Comparative Example 1 group did not exhibit a significant difference compared to the standard feed group, indicating that the ingestion of the whey proteins or Comparative Example 1 product could not suppress fat accumulation.
On the other hand, in the groups in which rats ingested Example 1 product or Example 2 product, the increase of the white adipose tissue weight was significantly lower compared to the standard feed group, whey protein group, and Comparative Example 1 group. Thus, the results demonstrated suppression of fat accumulation by ingestion of the fat accumulation suppressor of the present invention. These results suggest that the fat accumulation suppressor of the present invention provides an excellent fat accumulation suppressing effect and is useful for preventing an increase of myocardial infarction rate or mortality rate caused by obesity as well as for preventing a decline of quality of life.

### [Example 3]

### Production of tablets for suppressing fat accumulation

The ingredients were admixed according to the formulation shown in Table 4, and formed into 1 g tablets by a conventional method to produce tablets for suppressing fat accumulation according to the present invention.

**[Table 4]**

| | | |
|---|---|---|
| Hydrate crystalline glucose | 73.5 | (weight %) |
| Example 1 product | 20.0 | |
| Mineral mix | 5.0 | |
| Sugar ester | 1.0 | |
| Flavor | 0.5 | |

### [Example 4]

### Production of a nutritional composition for suppressing fat accumulation

500 g of Example 2 product was dissolved in 4500 g of deionized water. The mixture was heated to 50°C, and mixed by a TK homomixer (TK ROBO MICS; Tokushu Kika Kogyo) at 6000 rpm for 30 minutes to produce the solution A. To 5.0 kg of the solution A, 5.0 kg of casein, 5.0 kg of soybean proteins, 1.0 kg of fish oil, 3.0 kg of shiso oil, 18.0 kg of dextrin, 6.0 kg of mineral mix, 1.95 g of vitamin mix, 2.0 kg of emulsifier, 4.0 kg of stabilizer, and 0.05 kg of flavoring agent were added. The mixture was placed in 200 ml retort pouches and sterilized in a retort sterilizer (type 1 pressure vessel, TYPE: RCS-4CRTGN, Hisaka Works, Ltd.) at 121°C for 20 minutes. 50 kg of a nutritional composition for suppressing fat accumulation according to the present invention was thus produced.

### [Example 5]

### Preparation of a beverage for suppressing fat accumulation

30 g of powdered skim milk was dissolved in 670 g of deionized water, and 10 g of Example 1 product was further dissolved in the solution. The solution was heated to 50°C, and mixed by an Ultradisperser (ULTRA-TURRAX T-25; IKA Japan) at 9500 rpm for 30 minutes. 100 of maltitol, 2 g of acidulant, 20 g of reduced mizuame (starch syrup), 2g of flavoring agent, and 166 g of deionized water were added. The mixture was placed in 100 ml glass bottles and sterilized at 90°C for 15 minutes, and the bottles were sealed. Ten bottles (100 ml each) of a beverage for suppressing fat accumulation according to the present invention were thus produced.

## Claims

1. A fat accumulation suppressor comprising as an active ingredient a whey protein hydrolysate, wherein said hydrolysate has:
(1) a molecular weight range of no higher than 10 kDa and a main peak of molecular weight at 200 Da to 3 kDa;
(2) an APL (average peptide chain length) of 2 to 8;
(3) a free amino acid content of 20% or lower;
(4) a branched chain amino acid content of 20% or higher; and
(5) an antigenicity of 1/100,000 or lower relative to β-lactoglobulin.

2. The fat accumulation suppressor according to claim 1, wherein said hydrolysate is obtainable by a process comprising:
a step of hydrolyzing whey proteins with a use of a thermo-resistant protease enzyme while the whey proteins are heat-denatured at a temperature of 50 to 70°C and a pH of 6 to 10; and
a step of inactivating the enzyme by heating.

3. The fat accumulation suppressor according to claim 1, wherein said hydrolysate is obtainable by a process comprising:
a step of preliminarily hydrolyzing whey proteins with a use of a protease enzyme at a temperature of 20 to 55°C and a pH of 6 to 10;
a step of increasing the temperature to 50 to 70°C and hydrolyzing the whey proteins with a use of a thermo-resistant protease enzyme while the whey proteins are heat-denatured at a temperature of 50 to 70°C and a pH of 6 to 10; and
a step of inactivating the enzyme by heating.

4. The fat accumulation suppressor according to claim 1, wherein said hydrolysate is obtainable by a process comprising:
a step of preliminarily hydrolyzing whey proteins with a use of a protease enzyme at a temperature of 20 to 55°C and a pH of 6 to 10;
a step of increasing the temperature to 50 to 70°C and hydrolyzing the whey proteins with a use of a thermo-resistant protease enzyme while the whey proteins are heat-denatured at a temperature of 50 to 70°C and a pH of 6 to 10;
a step of inactivating the enzyme by heating;
a step of subjecting the inactivated reaction mixture to an ultrafiltration treatment with an ultrafiltration membrane having a molecular weight cut-off of 1 to 20 kDa; and
a step of subjecting a flow-through fraction from said ultrafiltration treatment to a microfiltration treatment with a microfiltration membrane having a molecular weight cut-off of 100 to 500 Da and collecting a non-flow-through fraction from said microfiltration treatment.

5. Food and beverage products, a nutritional composition, and animal feed for suppressing fat accumulation comprising the fat accumulation suppressor according to any of claims 1 to 4.

6. A method of producing a fat accumulation suppressor comprising:
a step of hydrolyzing whey proteins with a use of a thermo-resistant protease enzyme while said whey proteins are heat-denatured at a temperature of 50 to 70°C and a pH of 6 to 10; and
a step of inactivating the enzyme by heating.

7. The method according to claim 6, further comprising, prior to said hydrolysis step,
a step of preliminarily hydrolyzing whey proteins with a use of a protease enzyme at a temperature of 20 to 55°C and a pH of 6 to 10.

8. The method according to claim 6 or 7, further comprising, subsequent to said heat-inactivation step,
a step of subjecting the inactivated reaction mixture to an ultrafiltration treatment with an ultrafiltration membrane having a molecular weight cut-off of 1 to 20 kDa; and
a step of subjecting a flow-through fraction from said ultrafiltration treatment to a microfiltration treatment with a microfiltration membrane having a molecular weight cut-off of 100 to 500 Da and collecting a non-flow-through fraction from said microfiltration treatment.

9. A method of suppressing fat accumulation in a subject comprising administering to the subject 10 g/day or more of a whey protein hydrolysate, wherein said hydrolysate has:
(1) a molecular weight range of no higher than 10 kDa and a main peak of molecular weight at 200 Da to 3 kDa;
(2) an APL (average peptide chain length) of 2 to 8;
(3) a free amino acid content of 20% or lower;
(4) a branched chain amino acid content of 20% or higher; and
(5) an antigenicity of 1/100,000 or lower relative to β-lactoglobulin.
